# EUROPEAN PATENT SPECIFICATION

(11) **EP 2 704 730 B1**
(45) Date of publication and mention of the grant of the patent: **13.11.2019**
(21) Application number: 12779872.6
(22) Date of filing: 02.05.2012
(51) Int. Cl.: A61K 35/12, A61P 17/00, A61K 35/57

(54) **AVIAN-BASED TREATMENT FOR MICROBIAL INFECTIONS**
BEHANDLUNG MIKROBIELLER INFEKTIONEN MIT VOGELEXTRAKTEN
TRAITEMENT D'ORIGINE AVIAIRE POUR INFECTIONS MICROBIENNES

(30) Priority: 02.05.2011 AU 2011901619
(43) Date of publication of application: 12.03.2014
(73) Proprietor: Cocky Smart Pty Ltd, Crossman, WA 6390 (AU)
(72) Inventor: CHAMBERLAIN, John, CROSSMAN Western Australia 6390 (AU)
(74) Representative: AWA Sweden AB
(86) International application number: PCT/AU2012/000459
(87) International publication number: WO 2012/149600

(56) References cited:
- WO-A1-2008/019452
- WO-A1-2008/019453
- WO-A1-2008/019453
- WO-A2-2009/022842
- WO-A2-2009/057975
- US-A- 3 970 614
- US-A- 4 818 520
- J NAT ET AL: "Antibacterial activity of Pinus halepensis essential oil from Algeria(Tlemcen)", J. NAT. PROD. PLANT RESOUR, vol. 1, no. 1, 1 January 2011 (2011-01-01) , pages 33-36, XP055159166,
- GAURAV KAITHWAS ET AL: "Linum usitatissimum (linseed/flaxseed) fixed oil: antimicrobial activity and efficacy in bovine mastitis", INFLAMMOPHARMACOLOGY, KLUWER ACADEMIC PUBLISHERS, DORDRECHT, NL, vol. 19, no. 1, 1 February 2011 (2011-02-01), pages 45-52, XP002688296, ISSN: 0925-4692, DOI: 10.1007/S10787-010-0047-3
- RIGANO, L. ET AL.: 'Novel Retinol-Like Actives from Parrot Feathers' IFSCC MAGAZINE vol. 11, no. 8, 2008, pages 323 - 330, XP008110401
- BURTT JR, E.H. ET AL.: 'Colourful Parrot Feathers Resist Bacterial Degradation' BIOLOGY LETTERS. vol. 7, no. 2, 23 April 2011, pages 214 - 216, XP055134466

## Description

### Field of the Invention

The present invention relates to a composition and a kit for use in the treatment or prevention of a microbial skin infection.

### Background of the Invention

The skin has the largest surface area of all of the body organs and is the most exposed organ. Although the skin is remarkably effective in providing protection against the external environment, skin infections are nevertheless a common presentation in most family practices.

Bacteria can colonize the skin surface; both the outer stratum corneum and the underlying dermal layers to produce skin infections including erythrasma, impetigo, ecthyma, folliculitis, erysipelas and cellulitis. Bacteria commonly causing skin infections include Staphylococcus aureus, Streptococcus pyogenes, Corynebacterium minutissimum, Pasteurella multocida, Eikenella corrodens, and Bacteroides species. Viruses can also cause skin infections, such as the human papillomavirus (HPV) which causes focal areas of epithelial hyperplasia referred to as warts (including common warts, plantar warts, juvenile warts and condylomata). Indeed, selected types of HPV are capable of inducing malignant transformation of the epithelium, including that of the cervix. Fungi and yeast are also capable of causing many different forms of skin infections, broadly referred to as dermatomycoses. Candida albicans and Trichophyton, Epidermophyton, Microsporum and Malassezia spp are the most common infecting organisms and are often responsible for long and protracted infections resulting from their ability to permeate deep into the dermal layers. Infection with parasites such as those that cause scabies, mange or pediculosis can also colonise skin and hair follicles and cause much distress.

Skin infections may also result from a break in the integrity of the skin barrier, which allows the penetration of microorganisms through the normally protective skin layers to underlying dermal tissues. Bacteria such as Staphylococcus aureus, Pasteurella multocida, Eikenella corrodens, and Bacteroides species and viruses such as Haemophilus influenzae type B may gain entry through puncture wounds, insect bites or abrasions, potentially resulting in rapidly spreading and destructive cellulitis.

Although many treatments are available for skin infections, including physical means such as debridement or burning (through heat or cold) of warts, lancing of carbuncles and abscesses, antibiotics to treat bacterial and fungal infections, these have a number of problems, such as pain associated with physical treatments and side effects and resistance associated with antibiotics.

Accordingly, there is a need for novel pharmaceutical compositions containing bioactive substances which are able to treat or at least ameliorate microbial skin infections.

### Summary of the Invention

In one embodiment, the invention provides a composition comprising nicotinamide 1-10%, 2,4-ditert butyl phenol 1-10%, palmitic acid 5-40%, oleic acid 2-20%, and/or squalene 5-30% together with a pharmaceutically acceptable carrier and/or diluent for use in the treatment or prevention of microbial skin infections selected from the group consisting of: herpes oral cold sore (Type I), warts, impetigo, cellulitis, tinea, intertrigo, scabies, mange, pediculosis (including pediculosis capitis) cutaneous candidiasis or thrush.

According to a further embodiment, the invention provides a kit for use in the treatment or prevention of a microbial skin infection comprising: a composition comprising nicotinamide 1-10%, 2,4-ditert butyl phenol 1-10%, palmitic acid 5-40%, oleic acid 2-20%, and/or squalene 5-30% and a pharmaceutically acceptable carrier and/or diluent; and instructions regarding the administration of the composition for the treatment or prevention of a microbial skin infection, wherein the microbial skin infection is selected from the group consisting of: herpes oral cold sore (Type I), warts, impetigo, cellulitis, tinea, intertrigo, scabies, mange, pediculosis (including pediculosis capitis) cutaneous candidiasis or thrush.

A composition comprising an extract (hereafter known as EP-2) from the feathers of birds in a therapeutically effective concentration together with a pharmaceutically acceptable carrier for the use in treatment or prevention of a microbial skin infection is disclosed.

A method for the treatment or prevention of a microbial skin infection, wherein the method comprises administering to a subject in need thereof a composition comprising a therapeutically effective amount of EP-2 together with a pharmaceutically acceptable carrier and/or diluent is disclosed.

### Detailed Description of the Invention

### General

Those skilled in the art will appreciate that the invention described herein is susceptible to variations and modifications other than those specifically described herein and for the purpose of completeness these are included. The invention also includes all of the steps, features, compositions and compounds referred to or indicated in the specification, individually or collectively and any and all combinations or any two or more of the steps or features.

Functionally equivalent products, compositions and methods whether occurring naturally from birds or manufactured chemically to replicate the components of the avian extract are disclosed herein.

Throughout this specification, unless the context requires otherwise, the words "comprise", "comprises" or "comprising", will be understood to imply the inclusion of a stated integer or group of integers but not the exclusion of any other integer or group of integers.

Definitions for selected terms used herein may be found within the detailed description of the invention and apply throughout. Unless otherwise defined, all other scientific and technical terms used herein have the same meaning as commonly understood to one of ordinary skill in the art to which the invention belongs.

### Detailed Description of the Preferred Embodiments

It is disclosed that an extract from the skin and feathers of birds can be used to treat various microbial skin infections. It is further disclosed that a composition comprising an avian extract (hereafter known as EP-2) from the skin and feathers of birds in a therapeutically effective concentration together with a pharmaceutically acceptable carrier may be used in the treatment or prevention of a microbial skin infection. Reference to 'feathers' may be taken to mean 'feathers and skin' together, or 'feathers' or 'skin' individually. It is believed that the active components of the disclosed extract are provided on both the skin and the feathers of the bird and may spread from one area to another (e.g. from the skin of the bird along the feather shaft). The extract may be taken from whichever region of the bird is most convenient to collect from.

Whilst a similar extract has been disclosed by Chamberlain in WO 2008/019453 "Avian-based Insect Repellent", this extract was used solely as an insect repellent. The extract disclosed in WO 2008/019453 may be used in the treatment or prevention of a microbial skin infection such as a bacterial, fungal (including yeast), viral or parasitic skin infection.

Further, WO 2008/019452 relates to insect repellents comprising an effective amount of a phosphorous containing compound of the formula (RO)₃P=Oₓ or (RO)₂P=Oₓ(OH), alone or in synergistic combination with hydrocarbons, alkyl esters or dicarboxylic acids.

J NAT ET AL: "Antibacterial activity of Pinus halepensis essential oil from Algeria (Tlemcen)", J. NAT. PROD. PLANT RESOUR, vol. 1, no. 1, 1 January 2011 (2011 - 01-01), pages 33-36, XP055159166 relates to studies of antimicrobial activity of essential oil of Pinus halepensis tree against bacteria. The effect on four strains, including one strain of *Staphylococcus aureus*, one strain of *Bacillus cereus*, one strain of *Escherichia coli* and one strain of *Pseudomonas aeruginosa* were evaluated.

GAURAV KAITHWAS ET AL: "Linum usitatissimum (linseed/flaxseed) fixed oil: antimicrobial activity and efficacy in bovine mastitis", INFLAMMOPHARMACOLOGY, KLUWER ACADEMIC PUBLISHERS, DORDRECHT, NL, vol. 19, no. 1, 1 February 2011 (2011-02-01), pages 45-52, XP002688296, ISSN: 0925-4692, DOI: 10.1007/S10787-010-0047-3 relates to studies regarding the antimicrobial effect of *L. usitatissimum* fixed oil, which may be used in bovine mastitis, reducing the bacterial count in milk.

There is disclosed a method for treating or preventing microbial skin infections comprising the step of: administering to a subject in need thereof a composition comprising avian extract EP-2 in a therapeutically effective concentration together with a pharmaceutically acceptable carrier and/or diluent.

It is disclosed that the avian-based EP-2 extract may be obtained from birds of the order Psittaciformes (parrots). Suitable examples of such birds include Australian native parrots, such as the sulphur-crested cockatoo (*Cacatua galerita*), the galah (*Cacatua roseicapilla*), and the Major Mitchell cockatoo (*Cacatua leadbeateri leadbeateri*). It is further disclosed that the EP-2 may be obtained from the skin and feathers of such parrots, in particular tail feathers. It is further disclosed that the EP-2 may be obtained from the tail feathers of the sulphur-crested cockatoo.

The term "extract" as used herein refers to one or more compounds, typically in concentrated form, obtained by treating a material from which the extract is isolated with a solvent, after which the solvent is preferably removed. The term "extract" will also be understood to encompass one or more compounds obtained by subjecting a primary extract to subsequent purification processes known to those skilled in the art.

The avian-based extract may be extracted from bird feathers, in particular tail feathers, by treating the feathers with any one or more of a range of organic solvents. Suitable examples of organic solvents include, hydrocarbons (for example butane, pentane, hexane, heptane and octane); halogenated hydrocarbons (for example, methylene chloride, chloroform, trichloroethylene, carbon tetrachloride, trichloroethane, or trifluormethane); ethers (for example n-hexyl ether, methyl phenyl ether, ethyl phenyl ether and ethyl benzyl ether); ketones and aldehydes (for example acetone, acetonyl-acetone, benzaldehyde, acetophenone); alcohols such as ethyl alcohol, isopropanol; glycols such as diethylene glycol, ethylene glycol; and oils such as vegetable or mineral oils. The organic solvent can then be removed by techniques well understood to those skilled in the art, leaving a neat concentrate of EP-2.

Alternatively, EP-2 may be extracted derived from the tail feathers of a bird by aqueous extraction, any other form of solvent extraction or physical dusting.

The avian-based extract EP-2 may be extracted into 95% organic solvent. Solvents which may be used include Ethanol 95%, Dimethyl Sulfoxide (DMSO), or Dimethyl Formamide (DMF). Further, ethanol 95% may be used as the organic solvent.

The avian extract EP-2 may also be replicated in a synthetic form by combining two or more of the components of naturally occurring EP-2. This could be in the form of a single entity selected from the components of the avian extract, or a combination of two or more components. Preferably, the two or more components are in a synergistic relationship. The term "extract" will also be understood to mean any replicate formulation composed of one of more of the components that can be isolated from the avian extract or manufactured by chemical synthesis to resemble one of more of the compounds found in the extract. Thus, the term "extract" can be understood to mean the Synthetic Mixture.

For example the compounds TPP, Norpinene, Palmitic acid, Oleic acid, Benzoic acid, Napthalene, Nonanoinc acid, derivatives of Butylphenol, esters such as Hexadecanyl ester, phalates such as Iso- or Butyl- derivatives and Dioctadecyl phosphates have all been isolated as part of the EP-2 extract. Formulations that comprise combinations of one or more chemical equivalents which may be available commercially and therefore economically of advantage to use to deliver an equivalent therapeutically effective dose are also disclosed.

The Synthetic Mixture preferably comprises the following compounds: nicotinamide 1-10%, 2,4-ditert butyl phenol 1-10%, palmitic acid 5-40%, oleic acid 2-20%, and/or squalene 5-30%. Alternatively, the Synthetic Mixture may comprise the following compounds: nicotinamide at about 5.9%, 2,4-ditert butyl phenol at about 2.5%, palmitic acid at about 19.8%, oleic acid at about 7.6%, and/or squalene at about 12.1%.

The Synthetic Mixture may optionally also include BHT-aldehyde at about 0.05-2% and/or cholestenol at about 1-10%. Alternatively, the Synthetic Mixture may optionally comprise BHT-aldehyde at about 0.2% and/or cholestenol at about 4.0%.

The Synthetic Mixture can be dissolved in methanol as the solvent, or in water as the solvent to generate a solution for use as an extract.

It is disclosed that the avian extract EP-2 may be further processed into a powder form by, for example, air drying or freeze drying to remove moisture or the extraction solvent. Such dried compositions may be reconstituted in a suitable carrier or diluents for later application to the skin. It is disclosed that the extracted EP-2 may be dried under vacuum until less than 1% moisture remains. The extract may then be reconstituted using an organic solvent, for example Ethanol 95%, Dimethyl Sulfoxide (DMSO) or Dimethyl Formamide (DMF) which will aid in delivering an effective therapeutic dose. For example, DMSO is well known to those skilled in the art to aid in the penetration of topical therapeutics.

It is disclosed that the composition EP-2 may be used to treat or prevent a wide range of microbial skin infections.

Generally, the terms "treat", "treating" and "treatment" and derivatives used herein have the meaning to affect a subject, tissue or cell to produce a desired pharmacological and/or physiological effect. The treatment may be therapeutic in terms of a partial or complete resolution of the infection or the relief from the symptoms and clinical effects of the infection.

"Preventing" or "prevention" and derivative terms relate to the partial or complete prevention of a skin infection or its symptoms in a subject who: has not yet been diagnosed with a skin infection; has the skin infection on some but not all of their skin and wishes to prevent it developing on new regions; has had a skin infection and may be in remission and wishes to prevent re-occurrence; or has been diagnosed as being at risk of developing a skin infection.

For example, it is disclosed that the extract may be used in a method to treat skin infections caused by viruses, bacteria, parasites and/or fungi as either single causative agents or as combined causative agents in extreme skin infections.

It is disclosed a treatment using EP-2 of skin infections caused by a virus such as: Herpes virus (Types I and II), Human papillomavirus (HPV), poxviruses, Epstein-Barr, Herpes simplex virus, Molluscum contagiosum, Varicella viruses. Such virus may cause infections such as herpes cold sores (oral or genital), erythrasma, impetigo, ecthyma, folliculitis, erysipelas and cellulitis, warts (including common warts, Palmar, plantar, filiform, flat, refractory or periungual warts, or more specialized forms such as Bowenoid papulosis, Buschke-Löwenstein tumor, Butcher's (meat handler's) wart and condylomata), fifth disease, shingles, measles, roseola, rubella, German measles, chicken pox, Haemophilus influenzae type B or oral focal epithelial hyperplasia (Heck's disease).

It is disclosed that the skin infection to be treated with EP-2 is a type of herpes oral cold sore (Type I) or a type of wart.

It is disclosed that the extract may be used to treat or prevent infection by a bacterium. For example, EP-2 may be used to treat an infection by *Staphylococcus aureus*, other *Staphylococcus spp.* such as *S. epidermidis, Streptococcus spp* such as *S. pyogenes, Pasteurella multocida, Eikenella corrodens, Corynebacterium minutissimum, Bacteroides* species and *Corynebacterium spp.* These and other bacteria are responsible for infections such as cellulitis, folliculitis, furuncles, carbuncles, impetigo, erysipelas, and intertrigo.

It is disclosed that the extract may be used to treat or prevent impetigo or cellulitis.

It is disclosed that the avian EP-2 extract may be used to treat or prevent a microbial skin disease caused by infection with a fungal causative agent, including a yeast fungal agent. Such agents include the yeast *Candida albicans* and fungi such as *Trichophyton, Epidermophyton, Microsporum* and *Malassezi spp.* that cause dermatomycoses such as thrush, cutaneous candidiasis, tinea (including tinea versicolour; tinea cruris or jock itch; tinea capitis; tinea corporis or ringworm; tinea pedis or Athlete's foot) and intertrigo.

It is disclosed that the extract may be used to treat tinea, intertrigo, cutaneous candidiasis or thrush.

It is disclosed that the microbial infection to be treated by the extract may be a parasitic infection, such as those that cause scabies, mange or pediculosis (including pediculosis capitis).

It is disclosed that the EP-2 avian extract may be used to prevent the development of a microbial skin infection in a subject who has not been diagnosed with a microbial skin infection. Such subjects may, however, have been diagnosed as being at risk or susceptible to a microbial skin infection due to some other prevailing medical condition or event rendering them more susceptible. Alternatively, the subject may not be currently suffering from a microbial skin infection, but may suffer from recurring bouts of such infections and may wish to prevent the recurrence of another bout.

It is disclosed that the subject to be administered the composition may be a warm blooded vertebrate, for example a human, a companion animal such as dog or cat, domestic animal such as horse, cattle and sheep, or zoo animal such as non-human primate, canids, bovids, felids and ungulates. Alternatively, the subject in need of treatment may be avian. It is disclosed that the subject may be a human.

A composition comprising a therapeutically effective amount of avian extract EP-2 and one or more pharmaceutically acceptable additives, excipients carriers and/or diluents is disclosed. Further, the term "therapeutically effective amount" means an amount of EP-2 effective to yield a desired therapeutic response, for example to prevent or treat a microbial skin infection. The specific therapeutically effective amount will of course vary with such factors as the particular infection being treated, the physical condition and clinical history of the subject, the type of animal being treated, the duration of the treatment, the nature of concurrent therapy (if any), the specific formulations employed and the structure of the composition.

The term "pharmaceutically", "physiologically", or "veterinary acceptable" as used herein refers to pharmaceutically active agents, physiologically active agents, veterinary active agents, or inert ingredients which are suitable for use in contact with the skin of animals, including humans, without undue toxicity, incompatibility, instability, irritation, allergic response, commensurate with a reasonable benefit/risk ratio.

Additives, excipients carriers and diluents for use in the compositions of the present invention include, water, saline, ethanol, dextrose, glycerol, glycerol and polyhydric alcohols, milk protein, vitamins, animal and vegetable oils, polyethylene glycols, lactose, dextrose, sucrose sorbitol, mannitol and other sugars, starches, gum acacia, calcium phosphates, alginate, tragacanth, gelatine, calcium silicate, cellulose and its derivatives such as microcrystalline cellulose and methyl cellulose, polyvinylpyrrolidone, water syrup, methyl and propylhydroxybenzoates, talc, magnesium carbonate, titanium dioxide, magnesium stearate and mineral oil or combinations thereof.

The formulations can additionally include lubricating agents, dispersion media, pH buffering agents, wetting agents, emulsifying and suspending agents, solvents, preserving agents, sweetening agents or flavouring agents, antifoaming agents, polymers, antioxidants, chelating agents, viscomodulators, tonicifiers, flavorants, colorants, odorants, opacifiers, suspending agents, binders, fillers, plasticizers, lubricants, absorption-promoting agents and mixtures thereof. Preservatives include antimicrobial, antibacterial and antifungal agents, anti-oxidants, chelating agents and inert gases. The particular selection of constituent that can be included in the compositions described herein will generally depend on the type of preparation.

Suitable solvents include, ethyl alcohol, isopropanol, acetone, diethylene glycol, ethylene glycol, dimethyl sulfoxide, and dimethyl formamide. Suitable humectants include, acetyl arginine, algae extract, aloe barbadensis leaf extract, 2, 3-butanediol, chitosan lauroyl glycinate, diglycereth-7 malate, diglycerin, diglycol guanidine succinate, erythritol, fructose, glucose, glycerin, honey, hydrolyzed wheat protein/polyethylene glycol-20 acetate copolymer, hydroxypropyltπmonium hyaluronate, inositol, lactitol, maltitol, maltose, mannitol, mannose, methoxy polyethylene glycol, myristamidobutyl guanidine acetate, polyglyceryl sorbitol, potassium pyrollidone carboxylic acid (PCA), propylene glycol, sodium pyrollidone carboxylic acid.

It is disclosed that the EP-2 composition may further contain one or more additives, provided that they do not detrimentally affect the therapeutic effect afforded by the avian-based extract. The additive may be a colourant. Further, the additive may be a preservative such as a mould inhibitor or an anti-oxidant, a fragrance, or a stabiliser. In another embodiment the additive is a surfactant or an absorption-promoting agent such as dimethyl sulphoxide (DMSO). The additional additives may also be agents that render the composition an emulsion, a microemulsion or a nano-emulsion.

Additionally, the compositions may include surfactants or emulsifiers. Examples of surfactants include ethoxylated fatty acid glycerides, esters of fatty acids with polyols such as, for example, pentaerythritol or trimethylpropane, fatty alcohol ethoxylates or alkyl oligoglucosides, and electrolytes, such as sodium chloride and ammonium chloride. Examples of suitable emulsifiers include, stearic acid, cetyl alcohol, PEG-100, stearate and glyceryl stearate, cetearyl glucoside, polysorbate 20, methylcellulose, sodium carboxymethylcellulose, glycerin, bentonite, ceteareth-20, cetyl alcohol, cetearyl alcohol, lanolin alcohol, riconyl alcohol, self-emulsifying wax (e.g., Lipowax P), cetyl palmitate, stearyl alcohol, lecithin, hydrogenated lecithin, steareth-2, steareth-20, and polyglyceryl-2 stearate. Such surfactants and/or emulsifiers may be used for the formation of emulsions. Either a water-in-oil or oil-in-water emulsion may be formulated.

It is disclosed that the composition may be formulated to enable ready location and retention of the active ingredient(s) of the EP-2 extract in the area required to deliver the therapeutic effect while at the same time not significantly interfering with the efficacy of the EP-2 extract. It will be understood, therefore, that the therapeutically effective composition may be formulated differently based on the area of treatment and how the composition is to be applied.

There are a range of reference sources for the development of cosmetic and pharmaceutical compositions which may be referred to by the skilled person when developing formulations comprising EP-2, such as "A Formulary of Cosmetic Preparations; Volume 1 - Decorative Cosmetics", Hunting L.L. (2003); "A Formulary of Cosmetic Preparations; Volume 2 - Creams, Lotions and Milks", Hunting L.L. (2004) and "Remington's Pharmaceutical Sciences", 21st Edition (2009), Mack Publishing Company, Easton, Pennsylvania, USA, all the contents of which are incorporated herein. Other suitable guidebooks include Cosmetics and Toiletries Magazine, Vol. 111 (March, 1996); Formulary: Ideas for Personal Care; Croda, Inc, Parsippany, N.J. (1993); and Cosmeticon: Cosmetic Formulary, BASF.

It is disclosed that an avian extract EP-2 containing composition may be administered using standard procedures, for example: topically, parenterally, intraorbitally, ophthalmically, intraventricularly, intracranially, intracapsularly, intraspinally, intracisternally, intraperitoneally, buccally, rectally, vaginally, intranasally, orally or by aerosol administration and/or inhalation spray or via an implanted reservoir.

It is disclosed that the extract may be applied topically for use in the treatment of a microbial skin infection. The topical application may be in the form of directly laying or spreading the composition comprising the extract on the area of the proliferative skin disorder (or at least near or adjacent the proliferative skin disorder) using an applicator, such as a brush or a sponge, by spraying the composition directly onto the area, or by rubbing the composition onto the area. Alternatively, the EP-2 may be topically applied to an area of skin which does not yet suffer from a microbial skin infection.

Pharmaceutically acceptable carriers, adjuvants and vehicles that may be used in topical compositions may include: ion exchangers; alumina; aluminium stearate; lecithin; self-emulsifying drug delivery systems such as alpha-tocopherol polyethylene glycol 1000 succinate, or other similar polymeric delivery matrices or systems such as nanoparticles; serum proteins such as human serum albumin; buffer substances such as phosphates; glycine; sorbic acid; potassium sorbate; partial glyceride mixtures of saturated vegetable fatty acids; water; salts or electrolytes, such as protamine sulfate, disodium hydrogen phosphate, potassium hydrogen phosphate, sodium chloride, zinc salts; colloidal silica; magnesium trisilicate; polyvinyl pyrrolidone; cellulose-based substances; polyethylene glycol; sodium carboxymethylcellulose; polyacrylates; polyethylene-polyoxypropylene-block polymers; and wool fat.

The topical compositions may also be formulated with liquid or solid emollients, solvents, thickeners, or humectants. Emollients include, stearyl alcohol, mink oil, cetyl alcohol, oleyl alcohol, isopropyl laurate, polyethylene glycol, olive oil, petroleum jelly, palmitic acid, oleic acid, and myristyl myrstate. Emollients may also include natural butters extracted from various plants, trees, roots, or seeds. Examples of such butters include, shea butter, cocoa butter, avocado butter, aloe butter, coffee butter, mango butter, or combinations thereof. Suitable thickeners include, polysaccharides, in particular xantham gum, guar-guar, agar-agar, alginates, carboxymethylcellulose, relatively high molecular weight polyethylene glycol mono- and diesters of fatty acids, polyacrylates, polyvinyl alcohol and polyvinylpyrrolidone. The composition may further comprise one or more penetrants, compounds facilitating penetration of active ingredients into the skin of a patient. Examples of suitable penetrants include isopropanol, polyoxyethylene ethers, terpenes, cis-fatty acids (oleic acid, palmitoleic acid), acetone, laurocapram dimethyl sulfoxide, 2-pyrrolidone, oleyl alcohol, glyceryl-3- stearate, cholesterol, myristic acid isopropyl ester, and propylene glycol.

Compositions of the present invention may further comprise vitamins, anti-inflammatory agents, non-steroidal cosmetic soothing agents, skin lightening agents, anti-wrinkle agents, anti-itching agents, antioxidants, fragrances, conditioners, and other agents or any combination thereof. Those skilled in the art will readily appreciate that substances other than those listed may also be employed. Examples of useful vitamins include, vitamin A, vitamin B, biotin, vitamin C, pantothenic acid, vitamin K, vitamin D, vitamin E and mixtures thereof.

It is disclosed that a suitable vehicle for topical administration of EP-2 to the skin surface might include DMSO (dimethyl sulfoxide), ethanol, acetone, phosphatidyl choline and isopropanol gels. Additional additives may include a synergistic additive such as vitamin E to nutrify the skin, lanolin or aloe vera to soothe the skin. Other suitable additives would be known to those skilled in the art of pharmaceutical or cosmetic formulation.

The composition may be adapted for topical application and may be in a form selected from the group comprising cosmetically acceptable liquids, creams, oils, lotions, ointments, gels, roll-on liquids, skin patches, sprays, glass bead dressings, synthetic polymer dressings impregnated with basic milk factors, solids, conventional cosmetic night creams, foundation creams, suntan lotions, hand lotions, make-up, make-up bases and masks. It is disclosed that except insofar as any conventional medium or agent is incompatible with the active ingredient, use thereof in the cosmetic compositions may be contemplated. Compositions of the present invention adapted for topical delivery will desirably possess bioadhesive or mucoadhesive properties.

In another example, if the composition is used as a skin moisturiser it may preferably be of an ointment consistency. If the composition is used as a lip balm, the viscosity of the composition may preferably be increased to make a firmer product. The changes in viscosity of the composition may be achieved, for example, by adjusting the amount of beeswax. This and other methods will be familiar to those skilled in the art and further methods are described by Hunting, (2003); (2004) and Remington, (2009).

If the formulation is to be delivered as a spray or aerosol, the composition may also include a propellant. Preferably, hydrofluoroalkanes (HFA) such as either HFA 134a (1,1,1,2,- tetrafluoroethane) or HFA 227 (1,1,1,2,3,3,3-heptafluoropropane) or combinations of the two, may be used since they are widely used in medical applications . Other suitable propellants include, mixtures of volatile hydrocarbons, typically propane, n- butane and isobutane, dimethyl ether (DME), methylethyl ether, nitrous oxide, and carbon dioxide.

It is disclosed that the if the formulation is to be delivered topically, a range of different formulations may be developed to provide the avian extract EP-2 to the skin. For example, the EP-2 may be delivered as: a spray with ethanol or propylene glycol; a lotion with cetomacrogol lotion, aminobenzoic acid lotion or alcohol; a gel with poloxamer gel 8E, poloxamer gel 8C, chlorhexidine gel or Lutrol® F127; an ointment with liquid paraffin or white soft paraffin; or a buffered cream with emulsifying ointment, glycerol, cetomacrogol emulsifying wax 15 or propylene glycol.

Alternative formulations include gels, liquids, liquid solvents, dips, pastes, sprays, aerosols, and other solid formulations such as, for example, a wax-based solid. Other compositions can be formulated by those of ordinary skill in the art.

It is disclosed that the EP-2 extract may be formulated into:
- a spray for use in the treatment of microbial skin infections, comprising EP-2 extract qs together with 75% ethanol 95%, 5% propylene glycol and water to 100%; formulated in a fashion that will be familiar to those skilled in the art or as described in Remington, 2009. The pH and exact concentration of the various components of the composition are adjusted according to routine skills in the art.
- a lotion for use in the treatment of microbial skin infections, comprising EP-2 extract together with any one of the following: cetomacrogol lotion, aminobenzoic acid lotion or alcohol such as Lotion BPC containing EP-2 extract qs, 20% Glycerol, 60% Alcohol 95%, distilled water to 100%, with or without cetomacrogol emulsifying wax, aminobenzoic acid and/or liquid paraffin, formulated in a fashion that will be familiar to those skilled in the art or as described in Hunting, 2004 and Remington, 2009. The pH and exact concentration of the various components of the composition are adjusted according to routine skills in the art.
- a gel for use in the treatment of microbial skin infections, comprising EP-2 qs together with any one of the following: poloxamer gel 8E, poloxamer gel 8C, chlorhexidine gel or Lutrol® F127; formulated in a fashion that will be familiar to those skilled in the art or as described in Hunting, 2003; 2004 and Remington, 2009. The pH and exact concentration of the various components of the composition are adjusted according to routine skills in the art.
- an ointment for use in the treatment of microbial skin infections, comprising EP-2 qs, 30% emulsifying wax and 50% liquid paraffin or white soft paraffin formulated in a fashion that will be familiar to those skilled in the art or as described in Remington, 2009. The pH and exact concentration of the various components of the composition are adjusted according to routine skills in the art.
- an Aqueous Cream APF for use in the treatment of microbial skin infections, comprising EP-2 qs, 30% emulsifying ointment, 5% Glycerol, 1% Phenoxyethanol, 5% Vitamin E, and water to 100%, formulated in a fashion that will be familiar to those skilled in the art or as described in Hunting, 2004 and Remington, 2009. The pH and exact concentration of the various components of the composition are adjusted according to routine skills in the art.

Alternative carrier vehicles comprise gels, liquids, liquid solvents, dips, pastes, sprays, aerosols, and other solid formulations such as, for example, a wax-based solid. Other carrier vehicles can be formulated by those of ordinary skill in the art.

The composition may be adapted for personal care topical applications for use in the treatment or prevention of microbial skin infections. For example, the composition may be used by subjects who have a microbial skin infection and wish to treat it or who do not have a microbial skin infection but wish to prevent the occurrence of such an infection. The composition may also be used by subjects who have mild skin infections and wish to treat the infection prior to diagnosis. It is disclosed that subjects may find relief from a therapeutically effective amount of EP-2 extract delivered in any one of commonly used cosmetic or toiletry formulations which will be available to those skilled in the art. For subjects wishing to treat a microbial skin infection, it is disclosed that the EP-2 may be formulated into a composition adapted for cosmetic skin care, such as sun burn cream, gels lotion, makeup preparations, lotions, creams, sticks, roll-ons formulations, mousses, aerosol sprays, pad-applied formulations, and film-forming formulations as described by Hunting, 2003; 2004. The amount of EP-2 extract will be lesser than a prescriptive therapeutic does (for example between 1% and 50% volume/volume of the formulation). The precise concentration may be devised to suit the particular nature and extent of the infection and the causative agent.

It is disclosed that the extract may be delivered parenterally, preferably by injection, for example subcutaneously or intramuscularly. However, delivery may also be intra-arterially, intraperitoneally, intracavital, or intranasally for use in the treatment of a microbial skin infection.

Pharmaceutically acceptable carriers, adjuvants and vehicles that may be used in injection compositions and may include: ion exchangers; alumina; aluminium stearate; lecithin; self-emulsifying drug delivery systems (SEDDS) such as alpha-tocopherol polyethyleneglycol 1000 succinate, or other similar polymeric delivery matrices or systems such as nanoparticles; serum proteins such as human serum albumin; buffer substances such as phosphates; glycine; sorbic acid; potassium sorbate; partial glyceride mixtures of saturated vegetable fatty acids; water; salts or electrolytes, such as protamine sulfate, disodium hydrogen phosphate, potassium hydrogen phosphate, sodium chloride, zinc salts; colloidal silica; magnesium trisilicate; polyvinyl pyrrolidone; cellulose-based substances.

The formulation may be in the form of a sterile injectable preparation, for example, as a sterile injectable suspension for use in the treatment of proliferative skin conditions, including pre-cancers and skin cancer. This suspension may be formulated according to techniques known in the art using suitable dispersing agents, surfactants, and suspending agents (e.g. Tween 80). The sterile injectable preparation may also be a sterile injectable solution or suspension in a non-toxic parenterally-acceptable diluent or solvent (e.g. 1, 2-propanediol). Acceptable vehicles and solvents may include mannitol, water, Ringers solution and isotonic sodium chloride solution with buffer. Furthermore, sterile, fixed oils may be employed as a solvent or a suspending medium. For this purpose, any bland fixed oil may be employed including mono- or diglycerides. Fatty acids, such as oleic acid and its glyceride derivatives, and natural pharmaceutically acceptable oils, such as polyoxyethylated olive oil or castor oil, may also be used in the preparation of injectables.

It is disclosed that the avian extract EP-2 comprises about 0.0001% to 10% by weight of the composition administered to the subject having a microbial skin infection. However, the exact amount of EP-2 in the composition will of course, depend on the route of delivery, the nature of the infection, the therapeutically effective amount of EP-2 required and the general nature and health of the subject to whom the composition is delivered. In some cases, the amount of EP-2 in a composition may be 11-50%, or may be more than 50%, for example up to 60%, 70%, 80%, 90% or 95% of the composition. It is contemplated that in some instances the composition will comprise 100% EP-2, i.e. the avian extract will be administered directly to the subject as a neat extract.

Examples of suitable formulations that are disclosed include: a formulation comprising 50% volume 95% ethanol and 25% EP-2 and a combination of other ingredients to soothe the skin, emolliate the skin or 25% qs for topical application to the site of the microbial skin infection; or a formulation comprising 25% volume DMSO and 75% EP-2 for topical application to the site of the microbial skin infection.

It is disclosed that the concentration of the avian extract EP-2 will be of sufficient strength (qs) to provide a therapeutically effective dose to the target cells.

It is disclosed that the compositions comprising EP-2, may be adapted to be delivered to the subject in need once daily or less often. For example, if the composition is adapted for topical delivery, the composition may be administered to the skin once a day. However, it is contemplated that the topical composition can be administered more frequently than this (for example, two or three times a day). Alternatively, if the composition comprising EP-2 is in the form of an injectable formulation, it may be that the composition may be adapted for delivery once a week, or once a month.

A person with skill in the art will also know how to select a treatment regimen for a specific condition. For example, it is disclosed that the avian extract EP-2 may be applied once daily in topical form, or may be applied more frequently (for example, 2, 3 or 4 times daily). Alternatively, if the composition comprising EP-2 is in the form of an injectable formulation, it may be that the composition may be adapted for delivery once a week, or once a month. It is foreseeable that the composition may eb administered over a period of time. The composition may be applied for a day, multiple days, a week, multiple weeks, a month, or even multiple months in severe circumstances.

The use of avian extract EP-2 in a therapeutically effective concentration together with a pharmaceutically acceptable carrier and/or diluent in the manufacture of composition for use in the treatment or prevention of microbial skin infections is disclosed. The avian extract EP-2 may be isolated from tail feathers of a sulphur-crested cockatoo (Cacatua galerita). The avian extract EP-2 is a synthetic mixture comprising: nicotinamide 1-10%, 2,4-ditert butyl phenol 1-10%, palmitic acid 5-40%, oleic acid 2-20%, and/or squalene 5-30%.

A kit comprising a composition comprising EP-2 and instructions regarding the administration of the composition for use in the treatment or prevention of a microbial skin infection is disclosed. Further, the kit comprises a composition comprising EP-2 formulated for topical delivery and instructions regarding the topical application of the composition for use in the treatment of a microbial skin infection chosen from the list comprising: viral, bacterial, fungal or parasitic skin infections. Even further, the kit comprises a composition comprising EP-2 formulated for topical delivery and instructions regarding the topical application of the composition for the treatment of a microbial skin infection chosen from the list comprising: herpes oral cold sore (Type I), a type of wart, impetigo, cellulitis, tinea, thrush or intertrigo.

There is also disclosed a kit comprising a composition comprising EP-2 formulated for parenteral delivery and instructions regarding the parenteral delivery of the composition for use in the treatment of a microbial skin infection. Further, the kit comprises a composition comprising EP-2 formulated for parenteral delivery and instructions regarding the parenteral delivery of the composition for the treatment of a microbial skin infection chosen from the list comprising: viral, bacterial, fungal or parasitic skin infections. Even further, the kit comprises a composition comprising EP-2 formulated for parenteral delivery and instructions regarding the parenteral delivery of the composition for use in the treatment of a microbial skin infection chosen from the list comprising: herpes oral cold sore (Type I), a type of wart, impetigo, cellulitis, tinea, thrush or intertrigo.

The kits which are disclosed may comprise avian extract EP-2 isolated from tail feathers of a sulphur-crested cockatoo (Cacatua galerita) or alternatively avian extract EP-2 that is a synthetic mixture comprising: nicotinamide 1-10%, 2,4-ditert butyl phenol 1-10%, palmitic acid 5-40%, oleic acid 2-20%, and/or squalene 5-30%.

### Examples

The following examples are provided.

### Example 1

The effect of a synthetic representative chemical derived from the avian extract EP-2 (Triphenyl phosphate, TPP) was tested in vitro on *Staphyloccus aureus* and *Escherichia coli* cells as representative organisms capable of causing skin infection. TPP was investigated on the basis of the findings in Zurhaar WO2008/019452 "Insect Repellent" that the insect repellent factor in an avian extract was TPP.

A composition comprising 5% TPP, 20% pine oil, 50% raw linseed oil and 10% Recosol 150 in a 15% ethanol 95% carrier (w/w), was gently combined with an aqueous cream base containing vitamin E at 10% (w/w). The sample was then diluted in Tryptone Soya Broth (TSB), a broth that promotes the growth of microorganisms. Dilution concentrations of 01%, 0.2%, 0.4%, 0.8%, 1.6%, 3.1%, 6.3%, 12.5%, 25% and 50% product in TSB were tested. Dilutions were then inoculated with approximately 1 x 10⁶ colony forming units/ml and incubated for 48 hours at 37°C.

The TPP containing composition was found to completely inhibit the growth of *S. aureus* and *E. coli.* Growth of *S aureus* was inhibited by 50% composition in TSB (2.5% EP-2) whilst *E. coli* was inhibited by 3% composition (0.15% EP-2).

### Example 2

30% (w/w) of an EP-2 extract (extracted using ethanol) was combined in a base of natural oils in the approximate proportions of 50% (w/w) raw linseed oil and 20% (w/w) pine oil to make a lotion.

The lotion was administered to a 15 year old boy with a history of developing common warts which previous treatments available from pharmacy outlets had been unsuccessful in treating. During previous treatments, the extent of an individual wart was sometimes diminished but returned once the treatment was stopped. In other cases the individual warts were effectively unchanged by the treatment.

Treatment with the EP-2 lotion twice per day completely resolved the common warts within a seven day period and no return of the warts has occurred for at least 4 months.

### Example 3

Avian extract EP-2 has also been applied to Herpes I Type infections of lips representing as 'cold sores' in a patient with a history of cold sores, brought on by known stress triggers. Previously, over-the-counter medications containing 5% (w/w) acyclovir were used when cold sores had formed, or when an initial stage of their formation was detected (for example as a tingling sensation of the skin). When such OTC medication was applied topically, up to five times a day, the duration of the episode may well have been reduced as a result, but the patient still suffered unsightly and painful cold sores for several days.

In the present case, the lotion of Example 2 application was applied up to three times daily to an existing cold sore. Within a period of two days the cold sore blisters had resolved and were observed to slough off as a dried skin leaving behind a mild pink region. The pink region faded and disappeared within a five day period from the initial application.

Numerous variations and modifications will suggest themselves to persons skilled in the relevant art, in addition to those already described, without departing from the basic inventive concepts. All such variations and modifications are to be considered within the scope of the present invention, the nature of which is to be determined from the foregoing description.

sore blisters had resolved and were observed to slough off as a dried skin leaving behind a mild pink region. The pink region faded and disappeared within a five day period from the initial application.

Numerous variations and modifications will suggest themselves to persons skilled in the relevant art, in addition to those already described, without departing from the basic inventive concepts.

## Claims

1. A composition comprising nicotinamide 1-10%, 2,4-ditert butyl phenol 1-10%, palmitic acid 5-40%, oleic acid 2-20%, and/or squalene 5-30% together with a pharmaceutically acceptable carrier and/or diluent for use in the treatment or prevention of microbial skin infections selected from the group consisting of: herpes oral cold sore (Type I), warts, impetigo, cellulitis, tinea, intertrigo, scabies, mange, pediculosis (including pediculosis capitis) cutaneous candidiasis or thrush.

2. The composition for use according to claim 1, wherein the composition comprises: nicotinamide at 5.9%, 2,4-ditert butyl phenol at 2.5%, palmitic acid at 19.8%, oleic acid at 7.6%, and/or squalene at 12.1%.

3. The composition for use according to claim 1 or 2, wherein the composition further comprises: BHT-aldehyde at 0.05-2% and/or cholestenol at 1-10%.

4. A kit for use in the treatment or prevention of a microbial skin infection comprising: a composition comprising nicotinamide 1-10%, 2,4-ditert butyl phenol 1-10%, palmitic acid 5-40%, oleic acid 2-20%, and/or squalene 5-30% and a pharmaceutically acceptable carrier and/or diluent; and instructions regarding the administration of the composition for the treatment or prevention of a microbial skin infection, wherein the microbial skin infection is selected from the group consisting of: herpes oral cold sore (Type I), warts, impetigo, cellulitis, tinea, intertrigo, scabies, mange, pediculosis (including pediculosis capitis) cutaneous candidiasis or thrush.

## Patentansprüche

1. Zusammensetzung, umfassend 1-10% Nicotinamid, 1-10% 2,4-Di-tert.-butylphenol, 5-40% Palmitinsäure, 2-20% Ölsäure und/oder 5-30% Squalen zusammen mit einem pharmazeutisch unbedenklichen Träger und/oder Verdünnungsmittel zur Verwendung bei der Behandlung oder Prävention mikrobieller Hautinfektionen ausgewählt aus der Gruppe bestehend aus: oralen Herpesfieberbläschen (Typ I), Warzen, Impetigo, Zellulitis, Tinea, Intertrigo, Krätze, Räude, Pedikulose (einschließlich Pediculosis capitis), kutaner Kandidose oder Soor.

2. Zusammensetzung zur Verwendung nach Anspruch 1, wobei die Zusammensetzung Folgendes umfasst: 5,9% Nicotinamid, 2,5% 2,4-Di-tert.-butylphenol, 19,8% Palmitinsäure, 7,6% Ölsäure und/oder 12,1% Squalen.

3. Zusammensetzung zur Verwendung nach Anspruch 1 oder 2, wobei die Zusammensetzung weiterhin Folgendes umfasst: 0,05-2% BHT-Aldehyd und/oder 1-10% Cholestenol.

4. Kit zur Verwendung bei der Behandlung oder Prävention einer mikrobiellen Hautinfektion, umfassend: eine Zusammensetzung, die 1-10% Nicotinamid, 1-10% 2,4-Di-tert.-butylphenol, 5-40% Palmitinsäure, 2-20% Ölsäure und/oder 5-30% Squalen und einen pharmazeutisch unbedenklichen Träger und/oder ein pharmazeutisch unbedenkliches Verdünnungsmittel umfasst, und Anweisungen zur Verabreichung der Zusammensetzung bei der Behandlung oder Prävention einer mikrobiellen Hautinfektion, wobei die mikrobielle Hautinfektion ausgewählt ist aus der Gruppe bestehend aus: oralen Herpesfieberbläschen (Typ I), Warzen, Impetigo, Zellulitis, Tinea, Intertrigo, Krätze, Räude, Pedikulose (einschließlich Pediculosis capitis), kutaner Kandidose oder Soor.

## Revendications

1. Composition comprenant du nicotinamide 1 à 10 %, du 2,4-di-tert-butyl-phénol 1 à 10 %, de l'acide palmitique 5 à 40 %, de l'acide oléique 2 à 20 %, et/ou du squalène 5 à 30 % conjointement avec un support et/ou un diluant pharmaceutiquement acceptable pour une utilisation dans le traitement ou la prévention d'infections cutanées microbiennes choisies dans le groupe constitué par : herpès labial oral (type I), verrues, impétigo, cellulite, teigne, intertrigo, gale, pédiculose (y compris la pédiculose de la tête), candidose cutanée ou mycose.

2. Composition pour une utilisation selon la revendication 1, la composition comprenant : nicotinamide à raison de 5,9 %, du 2,4-di-tert-butyl-phénol à raison de 2,5 %, de l'acide palmitique à raison de 19,8 %, de l'acide oléique à raison de 7,6 %, et/ou du squalène à raison de 12,1 %.

3. Composition pour une utilisation selon la revendication 1 ou 2, la composition comprenant en outre : aldéhyde-BHT à raison de 0,05 à 2 % et/ou du cholesténol à raison de 1 à 10 %.

4. Kit pour une utilisation dans le traitement ou la prévention d'une infection cutanée microbienne comprenant : une composition comprenant du nicotinamide 1 à 10 %, du 2,4-di-tert-butyl-phénol 1 à 10 %, de l'acide palmitique 5 à 40 %, de l'acide oléique 2 à 20 %, et/ou du squalène 5 à 30 % et un support et/ou un diluant pharmaceutiquement acceptable ; et des instructions concernant l'administration de la composition pour le traitement ou la prévention d'une infection cutanée microbienne, l'infection cutanée microbienne étant choisie dans le groupe constitué par : herpès labial oral (type I), verrues, impétigo, cellulite, teigne, intertrigo, gale, pédiculose (y compris la pédiculose de la tête), candidose cutanée ou mycose.
